# NEW EUROPEAN PATENT SPECIFICATION

(11) **EP 2 553 425 B2**
(45) Date of publication and mention of the opposition decision: **01.03.2023**
(45) Mention of the grant of the patent: 28.11.2018
(21) Application number: 11715061.5
(22) Date of filing: 28.03.2011
(51) Int. Cl.: G01N 21/17, A61B 5/00, G01N 29/46, A61B 8/13, A61B 8/08

(54) **PHOTOACOUSTIC IMAGING APPARATUS AND PHOTOACOUSTIC IMAGING METHOD**
PHOTOAKUSTISCHE BILDGEBUNGSVORRICHTUNG UND PHOTOAKUSTISCHES BILDGEBUNGSVERFAHREN
APPAREIL D'IMAGERIE PHOTO-ACOUSTIQUE ET PROCÉDÉ D'IMAGERIE PHOTO-ACOUSTIQUE

(30) Priority: 02.04.2010 JP 2010086360
(43) Date of publication of application: 06.02.2013
(73) Proprietor: Canon Kabushiki Kaisha, Tokyo 146-8501 (JP)
(72) Inventor: FUKUTANI, Kazuhiko, Tokyo 146-8501 (JP)
(74) Representative: TBK
(86) International application number: PCT/JP2011/001821
(87) International publication number: WO 2011/121977

(56) References cited:
- EP-A2- 0 413 330
- GB-A- 2 444 078
- JP-A- H07 282 247
- US-A- 5 999 836
- US-A- 6 128 092
- US-A1- 2003 010 898
- US-A1- 2009 149 761
- US-A1- 2010 049 049
- F. J. Harris, ? On the Use of Windows for Harmonic Analysis with the Discrete Fourier Transform", PROCEEDINGS OF THE IEEE, VOL. 66, NO. 1, pages 51-83 (pages 172-204 of reprint), published in January 1978
- F. J. HARRIS: "On the Use of Windows for Harmonic Analysis with the Discrete Fourier Transform", PROCEEDINGS OF THE IEEE, vol. 66, no. 1, January 1978 (1978-01), pages 51-83, DOI: 10.1109/PROC.1978.10837
- M. XU et al.: "Universal back-projection algorithm for photoacoustic computed tomography", PHYSICAL REVIEW, vol. E 71, 016706, 19 January 2005 (2005-01-19), pages 1-7,

## Description

### Technical Field

The present invention relates to a photoacoustic imaging apparatus and a photoacoustic imaging method.

### Background Art

Research on optical imaging apparatuses for irradiating light onto a biological tissue from such a light source as a laser, and generating information on the biological tissue obtained based on the entered light as image data, are vigorously ongoing in medical fields. Photoacoustic imaging, including photoacoustic tomography (PAT), is one optical imaging technology. In the case of photoacoustic imaging, pulsed light generated from a light source is irradiated onto a biological tissue, and an acoustic wave (elastic wave, typically an ultrasound wave) generated from the biological tissue, which absorbed energy of the pulsed light which propagates and diffuses in the biological tissue, is detected at a plurality of positions. In other words, using the difference of absorptance of optical energy between an object area, such as a tumor, and another area of the tissue, an acoustic wave, which is generated when the object area is instantaneously expanded by absorbing irradiated optical energy, is received by a probe. By mathematically analyzing this detection signal, an optical characteristic distribution, particularly the absorption coefficient distribution in the biological tissue can be obtained. This information can be used for quantitatively measuring a specific substance in an object, such as glucose and hemoglobin contained in blood. Recently pre-clinical research for imaging blood vessels of small animals using the photoacoustic imaging, and clinical research for applying this theory to diagnosing breast cancer or the like, are making rapid progress (Non Patent Literature 1).

In photoacoustic imaging, a measurement performed in a state of a detection surface of an acoustic wave probe for detecting an acoustic wave and an area onto which the light is irradiated being on a same surface of the object is called reflection measurement (or reflection mode). In the case of reflection measurement, if light is irradiated onto an area directly underneath the probe in order to propagate the optical energy efficiently even to a deep area of the object, a large signal due to a photoacoustic wave generated by light absorption on the surface of the object directly underneath the measurement surface of the probe, is observed among the output signals from the probe. In this case, a signal, in which this signal and a photoacoustic signal generated from a light absorber inside the object are superposed, is observed, and as a result, an optical characteristic image of the light absorber deteriorates, which is a problem.

A method for solving this problem is written in Non Patent Literature 2. In Non Patent Literature 2, a dark-field illumination method, where light is irradiated from the sides of the probe without irradiating light directly underneath the probe, is used. According to this method, a large photoacoustic wave is not generated from the surface of the object directly underneath the detection surface of the probe, so the photoacoustic wave generated from the light absorber inside the object can be accurately measured, and image data of the light absorber inside the object can be generated without deteriorating the optical characteristic thereof.

### Citation List

### Non Patent Literature

NPL 1: "Photoacoustic imaging in biomedicine" M.Xu, L.V.Wang, REVIEW OF SCIENTIFIC INSTURUMENT, 77, 041101, 2006
NPL 2: "In vivo dark-field reflection-mode photoacoustic microscopy" K.Maslov, G.Stoica, L.V.Wang, Optics Letters, Vol.30, No.6, 625, 2005

Prior art which is related to this field of technology can be found in e.g. document GB 2 444 078 A disclosing an ultrasonic sensor which detects direct and reflected signals emanating from sample being imaged, in document US 6 128 092 A disclosing a method and system for high resolution ultrasonic imaging of small defects or anomalies, in document US 2003/0010898 A1 disclosing a system for measuring a biological parameter by means of photoacoustic interaction, and in document US 5 999 836 A disclosing enhanced high resolution breast imaging device and method utilizing non-ionizing radiation of narrow spectral bandwidth.

### Summary of Invention

### Technical Problem

However, in the case of Non Patent Literature 2, where light is not irradiated onto an area directly underneath the detection surface of the probe, it is difficult to efficiently propagate the light into the biological tissue, compared with the case of irradiating light onto an area directly underneath the detection surface of the probe. Therefore, an area (particularly an area in the depth direction) that can be imaged is limited.

With the foregoing in view, it is an object of the present invention to provide a technology to decrease the influence of the photoacoustic wave which is generated from the surface of an object in a photoacoustic imaging apparatus.

### Solution to Problem

This invention provides a photoacoustic imaging apparatus, a photoacoustic imaging method, and a photoacoustic imaging program according to the respective claims.

### Advantageous Effects of the Invention

According to the present invention, the influence of the photoacoustic wave generated from the surface of the object can be decreased in a photoacoustic imaging apparatus.

Further features of the present invention will become apparent from the following description of exemplary embodiments with reference to the attached drawings.

### Brief Description of Drawings

[fig. 1] Fig. 1 is a diagram depicting a configuration of photoacoustic imaging apparatus;
[fig.2]Fig. 2 is a flow chart depicting processing of detection signals;
[fig.3]Fig. 3 are diagrams depicting processing of detection signals;
[fig.4]Fig. 4 are diagrams depicting processing of a Fourier transform of Example 1;
[fig.5]Fig. 5 shows a configuration of a photoacoustic imaging apparatus of Example 1 and obtained images; and
[fig.6]Fig. 6 shows a configuration of a photoacoustic imaging apparatus of Example 2 and obtained images.

### Description of Embodiments

The present invention will now be described in detail with reference to the drawings. As a rule, the same composing elements are denoted with a same reference number, and redundant description thereof is omitted.

### (Photoacoustic imaging apparatus)

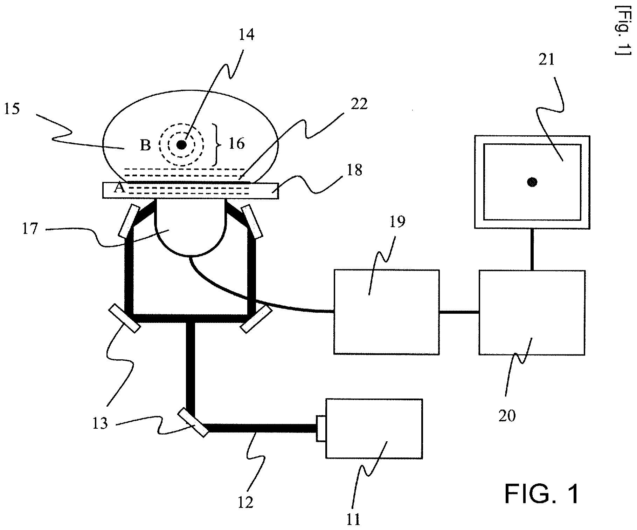

A configuration of a photoacoustic imaging apparatus of this embodiment will be described with reference to Fig. 1. The photoacoustic imaging apparatus of this embodiment is an apparatus for generating optical characteristic value information inside an object as image data. The optical characteristic value information generally refers to light absorption energy density distribution or absorption coefficient distribution.

As a basic hardware configuration, the photoacoustic imaging apparatus of this embodiment has a light source 11, an acoustic wave probe 17 as an acoustic wave detector, and a signal processor 20. A pulsed light 12 emitted from the light source 11 is guided by an optical system 13 which includes a lens, mirror, optical fiber and diffusion plate, for example, while being processed to be a desired light distribution profile, and is irradiated onto an object 15, such as a biological tissue. If a part of energy of light propagating inside the object 15 is absorbed by such a light absorber (which eventually becomes a sound source) 14 as a blood vessel, an acoustic wave (typically an ultrasound wave) 16 is generated by thermal expansion of the light absorber 14. This is also called a photoacoustic wave. The acoustic wave 16 is detected by the acoustic wave probe 17, amplified and converted into a digital signal by a signal collector 19, and then converted into image data of the object by the signal processor 20.

### (Light source 11)

The light source generates light to be irradiated onto an object. If the object is a biological tissue, light having a specific wavelength which is absorbed by a specific component, out of the components constituting the biological tissue, is irradiated from the light source 11. The light source may be integrated with the photoacoustic imaging apparatus of this embodiment, or may be disposed as a separate unit. For the light source, a pulsed light source which can generated pulsed light at a several nano to several hundred nano second order as the irradiation light is preferable. In concrete terms, about a 10 nano second pulse width is used in order to generated photoacoustic waves efficiently. Laser, which can implement large output, is preferable as a light source, but a light emitting diode or the like may be used instead of laser. For the laser, various lasers can be used, including a solid-state laser, gas laser, fiber laser, dye laser and semiconductor laser. The irradiation timing, waveform and intensity among others are controlled by a light source control unit, which is not illustrated.

In the present invention, it is preferable that a wavelength with which the light can propagate to an area inside the object is used if the object is biological tissue. In concrete terms, 500 nm or more, 1200 nm or less.

### (Optical system 13)

The light 12 irradiated from the light source 11 is guided to the object while being processed to be a desired light distribution profile typically by such optical components as a lens and mirror, but can also be propagated using such an optical waveguide as an optical fiber. The optical system 13 is, for example, a mirror for reflecting the irradiated light, a lens for collecting, expanding or changing the profile of the light, and a diffusion plate for diffusing the light. Any optical components can be used if the light 12 emitted from the light source can be irradiated onto the object 15 in a desired profile. In terms of safety of the biological tissue and a wider diagnostic area, it is preferable to expand the light to a certain area, rather than collecting the light by a lens.

In order to propagate the light energy to the object efficiently, it is preferable to use an optical system 13 which irradiates light onto the object surface 22 directly underneath the detection surface of the acoustic wave probe 17. In order to propagate more light energy to the object, it is preferable to use an optical system 13 which irradiates light onto the object in an object surface direction facing the acoustic wave probe 17. It is also preferable that the area for irradiating light onto the object is movable. In other words, it is preferable that the photoacoustic imaging apparatus is constructed so that the light generated from the light source is movable on the object. If movable, light can be irradiated in a wider range. It is more preferable that the area where light is irradiated onto the object (light to be irradiated onto the object) moves synchronizing with the acoustic wave probe 17. The method for moving the area where light is irradiated onto the object can be a method for using a movable mirror, or a method for mechanically moving the light source itself, for example.

### (Object 15 and light absorber 14)

These are not a part of the photoacoustic imaging apparatus, but will be described below. A major purpose of the photoacoustic imaging apparatus is diagnosing a malignant tumor and vascular disorders of humans and animals, and observing the progress of chemotherapy, for example. Therefore, an assumed object 15 is a biological tissue, in concrete terms a diagnostic target area such as the breast, finger, and limb of a human and animal body. A light absorber 14 inside the object is an area having a relatively high absorption coefficient in the object, and examples are oxidized hemoglobin or reduced hemoglobin, blood vessels containing a high level of both, or a malignant tumor containing many newly generated blood vessels, if the measurement target is a human body. Another example is a contrast medium which is injected in the body for contrasting a specific area, such as indocyanine green (ICG) and methylene blue (MB). An example of the light absorber on the object surface 22 is melanin existing around the surface of the skin. Hereafter biological information refers to a distribution of acoustic wave generation sources generated by light irradiation. In other words, biological information is an initial sound pressure distribution in the biological tissue, optical energy absorption density distribution derived therefrom, absorption coefficient distribution, and density distribution of a substance (particularly oxidized and reduced hemoglobin) constituting the biological tissue obtained from this information. An example of the density distribution of a substance is oxygen saturation. This biological information is generated as image data.

### (Acoustic wave probe 17)

The acoustic wave probe 17, which is a detector for detecting an acoustic wave generated on the surface of the object and inside the object by a pulsed light, detects an acoustic wave and converts the acoustic wave into electric signals, which are analog signals. Hereafter acoustic wave probe may simply be called a probe. Any acoustic wave detector may be used if an acoustic wave signal can be detected, such as a detector using piezoelectric phenomena, a detector using the resonance of light, and a detector using the change of capacitance. The probe 17 of this embodiment is typically a probe where a plurality of detecting elements are arrayed one-dimensionally or two-dimensionally. If such multi-dimensionally arrayed elements are used, an acoustic wave can be detected at a plurality of locations simultaneously, and the detection time can be shortened, and also the influence of vibrations of the object can be decreased.

### (Object surface flattening member 18)

According to this embodiment, it is preferable that the surface profile of the object 15 in the light irradiation area is flattened by disposing an object surface flattening member 18. If the light irradiation area of the object is already flat, the object surface flattening member 18 is unnecessary. In the case of not disposing the object surface flattening member 18, it is preferable that the acoustic wave probe 17 and the object 15 contact via such liquid as water or gel, so that the acoustic wave probe 17 and the object 15 receive the acoustic wave efficiently. Any member can be used for the object surface flattening member 18, if the member has a function to flatten the surface profile of the object. If the object surface flattening member 18 is disposed between the acoustic wave probe and the object, the probe and the object are acoustically coupled. In order to irradiate light onto a surface of an object immediately underneath the acoustic wave probe, a material, which is optically transparent so as to transmit the light and of which acoustic impedance is close to the object, is used. Typically in the case when the object is a biological tissue, polymethyl pentene, which is transparent and has an acoustic impedance close to the biological tissue, for example, is used. If light is irradiated onto the surface of the object facing the probe, the acoustic impedance need not be considered, therefore any optically transparent material that transmits light can be used, and typically such a plastic plate as acrylic or a glass plate can be used.

### (Signal collector 19)

It is preferable that the imaging apparatus of this embodiment has a signal collector 19 which amplifies an electric signal obtained by the probe 17, and converts the electric signal from an analog signal to a digital signal. Typically the signal collector 19 is constituted by an amplifier, A/D convertor, and an FPGA (Field Programmable Gate Array) chip among others. If a plurality of detection signals are obtained from the probe, it is preferable that a plurality of signals can be simultaneously processed. Thereby time to generate an image can be decreased. In this description, detection signal is a concept including both an analog signal obtained from the probe 17 and a digital signal after this analog signal is A/D-converted. The detection signal is also called a photoacoustic signal.

### (Signal processor 20)

The signal processor 20 performs processing to reduce photoacoustic wave signals generated on the surface of the object, which is a characteristic processing of the present invention. Then the signal processor 20 generates and obtains image data inside the object using the detected signals obtained after the reduction processing. Though details are described later, characteristic of the present invention is that the processing to reduce photoacoustic wave signals generated on the surface of the object is performed, using the difference of characteristics between the photoacoustic wave signal generated on the surface of the object and the photoacoustic wave generated from the light absorber inside the object.

For the signal processor 20, a workstation or the like is normally used, so as to perform processing to reduce photoacoustic wave signals generated on the surface of the object, and image reconstruction processing or the like, by pre-programmed software. For example, a software running on a workstation has two modules: a signal processing module for performing processing to reduce photoacoustic wave signals generated on the surface of the object, and noise reduction processing; and an image reconstruction module for reconstructing an image to generate image data. In the photoacoustic imaging, the noise reduction processing among other processings is normally performed on a signal received at each position as preprocessing before reconstructing the image, and it is preferable that these processings are performed in the signal processing module. In the image reconstruction module, image data is generated by image reconstruction, and inverse projection in the time domain or Fourier domain, for example, which is normally used in tomography technology, is performed as an image reconstruction algorithm. If it is possible to spend time for image reconstruction, such an image reconstruction method as an inverse problem analysis method using a repetitive processing can also be used. As Non Patent Literature 2 shows, typical examples of the image reconstruction method of PAT are: Fourier transform method, universal back projection method and filtered back projection method. In order to decrease the image reconstruction time, it is preferable to use a GPU (Graphics Processing Unit) installed in a workstation, that is the signal processor 20. If the receive signal is already in proportion to the image in the depth direction using a focus type acoustic wave probe of which observation points are limited, for example, image reconstruction is unnecessary, and receive signals may be directly converted into image data. Such processing is also performed in the image reconstruction module.

The signal collector 19 and the signal processor 20 may be integrated. In this case, the image data of the object may be generated by hardware processing, instead of the software processing executed on a workstation.

It can be said that the signal processor 20 is a combination of a Fourier transformer, which performs Fourier transform, and an inverse-Fourier transformer, which reduces or removes components less than or equal to a predetermined frequency, and performs inverse-Fourier transform to return the signal back to a time signal (corresponds to the second signal of the present invention). If the signal processor 20 is implemented as software, it can be regarded that the Fourier transformer and the inverse-Fourier transformer correspond to each function of a module.

### (Display 21)

The display 21 is an apparatus for displaying images based on the image data which is output by the signal processor 20, and typically a liquid crystal display is used. The display may be provided separately from the photoacoustic image diagnostic apparatus.

### (Detection signal processing)

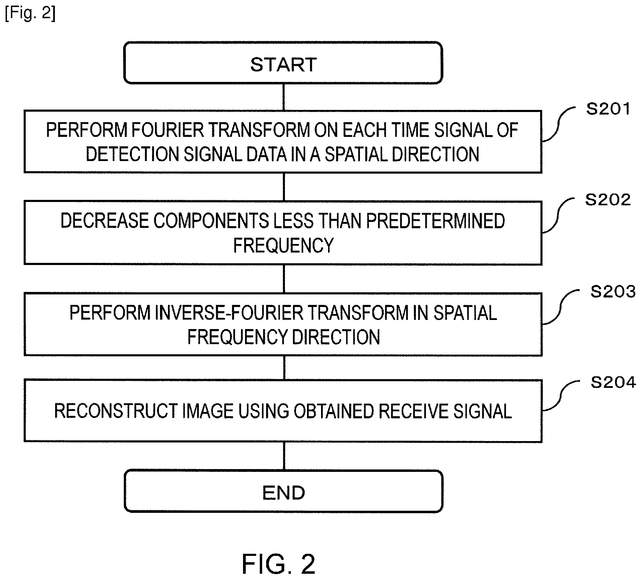

Processing for the signal processor 20 to reduce photoacoustic wave signals generated on the surface of the object, which is a characteristic of the present invention, will now be described with reference to Fig. 2, Fig. 3 and Fig. 4. Step numbers in the following description correspond to the step numbers in the flow chart in Fig. 2.

### Processing [1] (step S201): step of performing Fourier transform on the detection signal data in a spatial direction (array direction of the detecting elements).

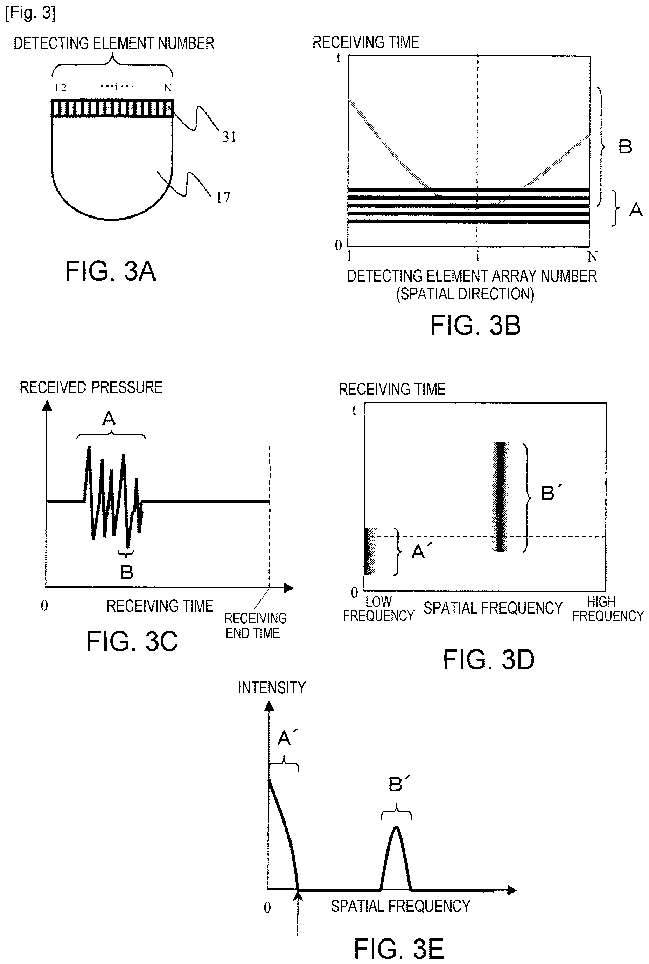

Fourier transform is performed on digital signals obtained from the signal collector 19 shown in Fig. 1 at the same detection time, in a spatial direction, that is the array direction of the acoustic wave detecting elements. Here a case of a one-dimensional array probe, as shown in Fig. 3A, will be described as an example. First each detection signal data obtained by each detecting (receiving) element 31 is mapped with the detecting element numbers (e.g. 1 to N) on the abscissa, and the receiving time (e.g. 0 sec. to t sec.) on the ordinate, so as to generate two-dimensional array data of which level is the received sound pressure value. Fig. 3B is an image of the two-dimensional array data, and the brightness indicates the level of received sound pressure value (black indicates an area where received sound pressure is high). The receiving time here means the time from receiving start time, which is light irradiation until the end of receiving the photoacoustic waves generated from the area inside the object by the detecting elements.

Fig. 3C shows an example of a detection signal of a detecting element (i-th) in the location of the dotted line in Fig. 3B. In Fig. 3C, the abscissa is the receiving time, and the ordinate is the received sound pressure. Normally if a pulsed light is irradiated onto the object 15, a plurality of N-shaped sound pressure signals are observed as detection signals, as shown in Fig. 3C. The point when the receiving time is zero is the time the pulse light is irradiated. These N type signals are mainly detection signals by photoacoustic waves, which are generated from a light absorber 14 (e.g. blood in the case of a biological tissue) inside the object, and a surface of the object (e.g. pigments on the surface of skin in the case of a biological tissue). The sound pressure of the photoacoustic wave generated on the surface of the object is generally higher (larger) than the sound pressure of the photoacoustic wave generated from the light absorber inside the object. The reason why a relatively high (large) photoacoustic wave is generated on the surface of the object onto which light is irradiated is because the intensity of light to be irradiated onto the surface is higher than an area inside the object, even if the light absorption coefficient of the surface of the object itself is smaller than that of the light absorber inside the object.

In the example in Fig. 3C, A denotes a detection signal area due to the photoacoustic wave generated on the surface 22 of the object directly underneath the detection surface of the probe, and B denotes a detection signal area due to the photoacoustic wave generated from the light absorber 14 inside the object. In Fig. 1 as well, A denotes a photoacoustic wave generated from the surface of the object, and B denotes a photoacoustic wave generated from the light absorber inside the object. As Fig. 3C shows, if the photoacoustic wave B from the light absorber inside the object is received while receiving the photoacoustic wave A, it becomes difficult to distinguish photoacoustic wave A and photoacoustic wave B from each other. As a result, obtaining a desired image becomes difficult. This will be described using images in Example 1 and Example 2.

In the above mentioned Fig. 3B as well, the time domain A indicates the time of receiving the detection signals due to the photoacoustic wave generated on the surface of the object, and the time domain B indicates the time of receiving the detection signals due to the photoacoustic wave generated from the light absorber 14 inside the object. Whereas the detection signals of the photoacoustic wave A in Fig. 3B are detected by each detecting element almost at the same time, and the detection time of the detection signals of the photoacoustic wave B is different depending on the detecting element. The reason for this will now be described. As Fig. 1 shows, if light is uniformly irradiated with setting the surface of the object to be parallel with the receiving surface of the probe by arranging an object surface flattening member 18, for example, the photoacoustic wave generated from the surface of the object propagates like a plane wave, as shown in A in Fig. 1, and is received by the probe 17. The photoacoustic wave 16, on the other hand, propagates like a spherical wave in many cases, as shown in B in Fig. 1, and is received by the probe 17, since the light absorber inside the object is sufficiently smaller than the light irradiation area. Because of this difference in the propagation characteristics, the photoacoustic signal having the characteristics shown in Fig. 3B is obtained.

In the normal photoacoustic imaging, the intensity (brightness) of the photoacoustic wave A in Fig. 3B depends on the distribution of the light irradiated onto the surface of the object. In other words, the magnitude of the detection signal in A is not constant in a same receiving time, but a sound pressure of which level is in proportion to the light irradiation distribution intensity is detected. Therefore, in order to unify the receiving intensity at a same receiving time in A in Fig. 3B, it is preferable to normalize the detection signal by each detecting element with the intensity distribution of the light irradiated onto the object. In other words, it is preferable to perform such processing as multiplying each detection signal by an inverse number of the light intensity distribution.

In the present invention, Fourier transform is performed on each receive data at a same receiving time in the array direction of the detecting elements, so as to generate two-dimensional spatial frequency data. Fig. 3D shows an image of the two-dimensional spatial frequency data generated by plotting spatial frequency on the abscissa, and the receiving time on the ordinate, regarding the brightness as the intensity of frequency components. If the detecting elements of the probe are arrayed two-dimensionally, Fourier transform (two-dimensional Fourier transform) may be performed for arrays in each direction, or the two-dimensional array may be arranged one-dimensionally so that Fourier transform is performed in this array direction. In Fig. 3D, A' is the characteristic frequency components of the photoacoustic wave generated from the surface of the object, and B' is the characteristic frequency components generated from the light absorber inside the object.

Fig. 3E is a graph plotting the data of the area of the broken line in Fig. 3D at a same receiving time. In Fig. 3E, the ordinate is the intensity of each frequency component, and the abscissa is the spatial frequency. In Fig. 3E as well, A' denotes the characteristic frequency components of the photoacoustic wave generated from the surface of the object, and B' denotes the characteristic frequency components of the photoacoustic wave generated from the light absorber inside the object. As the examples in Fig. 3D and Fig. 3E show, the detection signals of the photoacoustic wave generated from the surface of the object are detected by each detector at the same time. In other words, a signal having a same magnitude is received at a same receiving time, so the spatial frequency signals in the array direction of the detecting elements include many low frequency component signals, of which main components are DC components. The detection signals of the photoacoustic wave generated from the light absorber inside the object, on the other hand, include many high frequency components compared with the above mentioned case, since the receiving time is different depending on the element. In other words, if Fourier transform is performed, in the spatial direction, on the detection signals, the receiving time domain A of the photoacoustic wave generated from the surface of the object and the receiving time domain B of the photoacoustic wave generated inside the object can be clearly distinguished.

### Processing [2] (step S202): step of reducing the frequency components (predetermined frequency components), due to the detection signals of the photoacoustic wave generated from the surface of the object, in the spatial frequency signals.

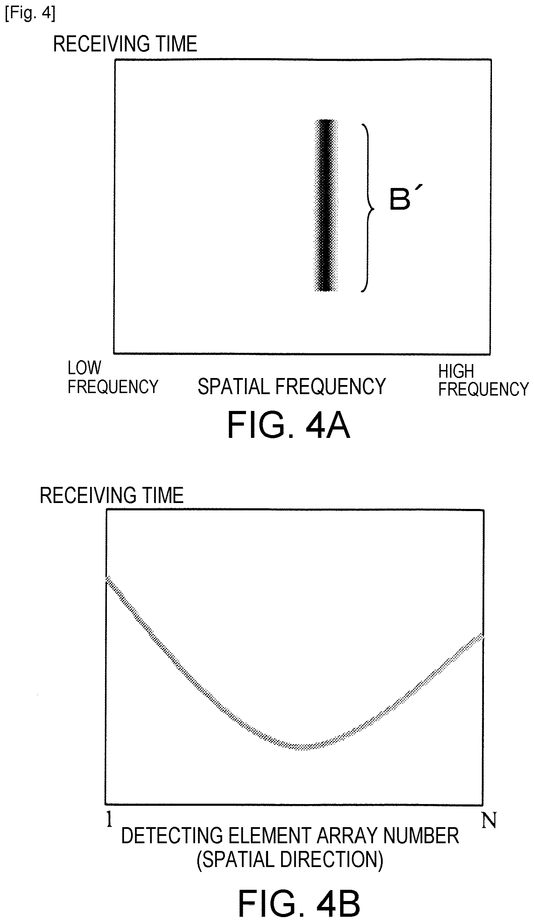

In this processing, A' in Fig. 3D is eliminated in the spatial frequency signals obtained by the above mentioned processing, so as to generate the signals shown in Fig. 4A. The value of the signal area of A' here is sufficiently smaller than the value of the signal area of B', therefore it can be zero or simply be decreased to a small value. The main components of the frequency components, due to the detection signals of the photoacoustic wave generated from the surface of the object, are DC components. Therefore, the predetermined frequency components to be reduced in the present invention are DC components. In actual detection signals, however, not only DC components but also low frequency components are included, as shown in Fig. 3E, because of the influence of the light irradiation distribution or the like. Hence the predetermined frequencies to be reduced in this invention are the frequency components at frequencies up to the point indicated by the arrow mark in Fig. 3E. This means that the frequencies up to the point shown by the arrow mark indicate the predetermined frequencies according to the present invention. For example, if d is the length of the detector in the array direction of the detecting elements (probe width), the spatial frequency f of the fundamental wave is f = 1/d, and nf is a frequency of the n-th harmonic wave (n is an integer), then frequencies less than the predetermined frequencies according to the present invention refers to the DC components and the frequency components of the n-th harmonic wave. The value n depends on the apparatus configuration, such as the light irradiation distribution, therefore it cannot be defined. This means that the value n is a parameter unique to the apparatus. Hence it is preferable to determine the value n based on experience, by analyzing the signals obtained from the apparatus.

### Processing [3] (step S203): step of performing inverse Fourier transform on the signal obtained in processing [2] in the spatial frequency direction, and converting it into a time signal.

Inverse Fourier transform is performed on the spatial frequency signals at a same receiving time obtained in the processing [2] in the frequency direction. For example, inverse Fourier transform is performed in Fig. 4A in the frequency direction, then [Fig. 4A] is converted into a second detection signals, as shown in Fig. 4B. As the comparison of Fig. 3B and Fig. 4B show, which are states before and after the processing of the present invention, the detection signals of the photoacoustic wave generated from the surface of the object have been decreased. If the detection signal is normalized with the light irradiation intensity in processing [1], it is preferable to multiply the obtained second detection signals by light intensity to convert them into the original detection signal value area.

### Processing [4] (step S204): step of generating image data inside the object using the processed detection signals.

Image construction processing is performed using the digital detection signal data obtained in processing [3], so as to generate image data related to the optical characteristic value distribution of the object. In this case, if the signals in which the detection signals of the photoacoustic wave generated on the surface of the object are decreased are used, as shown in Fig. 4B, then only image data on the light absorber inside the object can be generated, and a diagnostic image can be created without image deterioration. Any image reconstruction method may be used, but normally an inverse projection in time domain or Fourier domain, which is used in generation photoacoustic imaging, for example, is used (see Non Patent Literature 2). As mentioned above, if the image reconstruction processing is unnecessary, the digital detection signal data obtained in processing [3] is directly converted into an image.

By performing the above steps, only the detection signals of the photoacoustic wave generated from the surface of the object can be reduced, and by using the detection signals generated after this reduction processing for the image reconstruction, image data can be generated without deteriorating the optical characteristic value distribution of the light absorber inside the object.

### <Example 1>

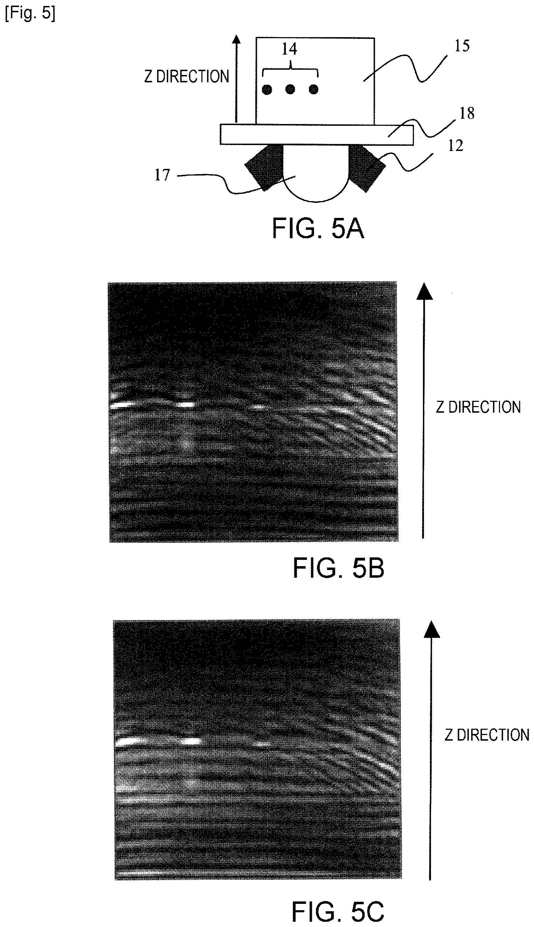

An example of a photoacoustic imaging apparatus to which this embodiment is applied will be described. The schematic diagrams in Fig. 1 and Fig. 5A are used for description. In this example, a Q switch YAG laser, which generates about a 10 nano second pulsed light at wavelength 1064 nm, is used for the light source 11. The energy of the optical pulse emitted from the pulsed laser beam 12 is 0.6 J. An optical system 13 is set such that after expanding the pulsed light up to about a 1 cm radius using the optical system 13 of a mirror, beam expander and the like, the pulsed light is split into two by a beam splitter, and the lights are irradiated onto the object directly underneath the probe.

For the object 15, a rectangular phantom simulating a biological tissue, as shown in Fig. 5A, is used. The phantom used here is a 1 percent Intralipid solidified by agar-agar. The size of the phantom is 6 cm width, 6 cm height and 5 cm depth. In this phantom, as shown in Fig. 5A, three objects, which are solidified to be 2 mm diameter cylinders and are colored with ink, are embedded around the center as the light absorber 14. After flattening the light irradiation surface 22 of the phantom by a 1 cm thick plate type member 18 constituted by polyethylpentene, the probe is contacted via the plate 18. Gel is applied between the plate 18 and the probe, and between the phantom and the plate for acoustic matching. In the phantom being set like this, the pulsed light 12 is irradiated onto the surface of the phantom directly underneath the probe 17. For the acoustic wave probe 17, a probe made from PZT (lead zirconate titanate) is used. This probe is a two-dimensional array type, of which a number of elements is 324 (18 * 18), and the element pitch is 2 mm in each direction. The size of the element is about 2 * 2 mm².

As Fig. 1 and Fig. 5A show, if the pulsed light 12 is irradiated onto the surface of the phantom directly underneath the probe 17, a photoacoustic wave, due to light absorption by the surface of the phantom on the light irradiation side and a photoacoustic wave due to the cylindrical light absorber 14 absorbing the light diffused in the phantom are generated. These photoacoustic waves are received by the probe 17 via 324 channels at the same time, and the detection signals are obtained using the signal collector 19 constituted by the amplifier, A/D converter and FPGA, so as to obtain digital data of the photoacoustic signals in all the channels. In order to improve the S/N ratio of the signals, laser is irradiated 30 times, and the time values of all the obtained detection signals are averaged. The obtained digital data is then transferred to the work station (WS) which is the signal processor 20, and is stored in the WS.

Then based on the stored receive data, a three-dimensional array signals are generated by plotting the element numbers in the probe array directions in the X and Y axes and the receiving time in the Z axis. Two-dimensional Fourier transform is performed on the three-dimensional array data for each receiving time in the element array directions, so as to generated three-dimensional spatial frequency data.

After the values of the first three points on the low frequency side of the spatial frequency signals at each receiving time are set to zero, two-dimensional inverse Fourier transform is performed, and the result is converted into three-dimensionally arrayed detection signal data again, by plotting the element numbers in the array directions in the X and Y axes and the receiving time in the Z axis. Then the image is reconstructed using this data. Here three-dimensional volume data is generated using a universal back projection method, which is a time domain method. The voxel interval used here is 0.05 cm. The imaging range is 3.6 cm * 3.6 cm * 4.0 cm. Fig. 5B shows an example of an image (tomographic image) obtained in this case.

On the other hand, an image is reconstructed directly using the detection signal data stored in the WS, without reducing the detection signals of the photoacoustic wave generated from the surface of the object. Fig. 5C shows an example of an image (tomographic image) obtained in this case. Both Fig. 5B and Fig. 5C show a two-dimensional cross-section near the center of the phantom.

Fig. 5B and Fig. 5C are compared. In Fig. 5C, a signals due to the photoacoustic wave generated on the surface of the phantom, because of multiple reflection and other reasons, are detected at a plurality of points in receiving time, and as a result, linear images appear at various locations in the depth direction (Z direction). In Fig. 5B, on the other hand, the signals received due to the acoustic wave generated on the surface of the phantom are reduced, therefore the images due to the photoacoustic signals are reduced, and the image of the light absorber inside the phantom is displayed more clearly than in Fig. 5C. In this way, by reducing the receive data due to the photoacoustic signals generated from the surface of the object, the image of the light absorber inside the object can be generated without deteriorating the image.

### <Example 2>

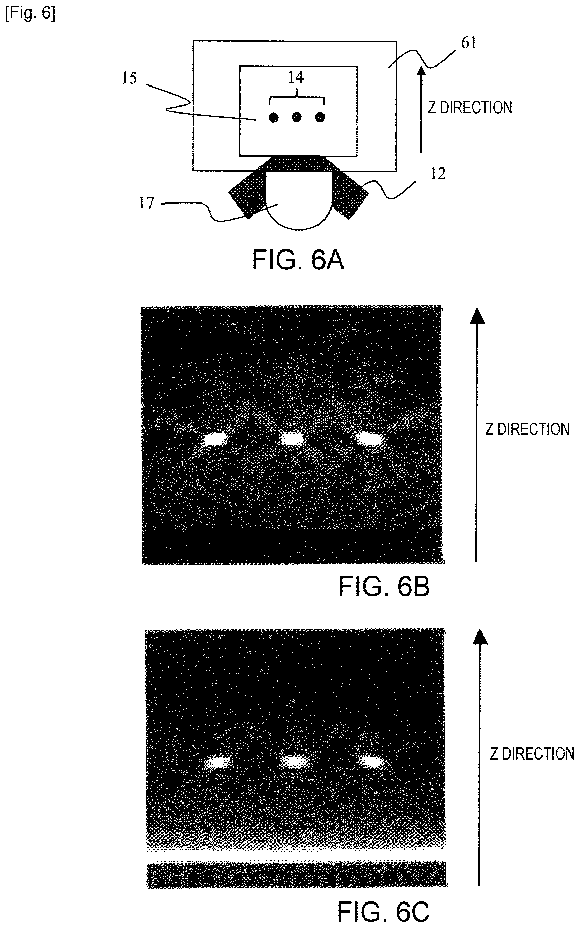

A case of the photoacoustic imaging apparatus which does not require the object flattening member 18 will be described as Example 2, with reference to Fig. 6A. The basic configuration of this example is the same as Example 1, but the object flattening member 18 does not exist between the probe 17 and the object 15.

For the object 15, a phantom simulating a biological tissue is used. The phantom used here is generally the same as Example 1. In order to acoustically match with the acoustic wave probe 17, the phantom is set in a tank 61 filled with water, so as to contact the probe 17 via water. In the phantom being set like this, the pulsed light 12 is irradiated onto the surface of the phantom directly under the detection surface of the probe 17. For the acoustic wave probe 17, a probe the same as Example 1 is used. Then the intensity distribution of the light irradiated onto the phantom is measured and stored in the WS, which is a signal processor. The photoacoustic wave generated by light irradiation is received by the probe, just like Example 1, and the obtained digital data is stored in the WS. The stored receive data is normalized with the irradiation distribution of the light irradiated onto the phantom.

Then the same processing as Example 1 is performed on the normalized data, and the signal data, in which the detection signals due to the photoacoustic wave generated from the surface of the object are reduced, is generated. After this signal data is multiplied by the light irradiation distribution, an image is reconstructed just like Example 1, and volume data is generated. Fig. 6B shows an example of the tomographic image obtained in this case. On the other hand, an image is reconstructed again directly using the detection signal data stored in the WS, without reducing the detection signals of the photoacoustic wave generated from the surface of the object. Fig. 6C shows an example of the tomographic image obtained in this case.

Fig. 6B and Fig. 6C are compared. In Fig. 6C, linear images generated based on the detection signals due to the photoacoustic wave generated on the surface of the phantom are clearly displayed. In Fig. 6B, on the other hand, the signals received due to the photoacoustic wave generated on the surface of the phantom are reduced, therefore the images due to the signals are reduced, and the image of the light absorber inside the phantom is displayed more clearly than above-mentioned Fig. 5B. In this way, by reducing the receive data due to the photoacoustic signals generated from the surface of the object from the detection signals normalized with light intensity distribution, the image of the light absorber inside the object can be generated without deteriorating the image.

Within the scope of the appended claims, the present invention can be embodied in various modes, without being limited to the above examples. For example, the present invention can be regarded as a photoacoustic imaging method for each composing element of the apparatus irradiates light and detect signals, and for the information processor (signal processor) to generate image data. The present invention can also be regarded as a photoacoustic imaging program for controlling each composing element of the apparatus, and having the information processor generate image data.

While the present invention has been described with reference to exemplary embodiments, it is to be understood that the invention is not limited to the disclosed exemplary embodiments. The scope of the following claims is to be accorded the broadest interpretation so as to encompass all such modifications, structures and functions.

This application claims the benefit of Japanese Patent Application No. 2010-086360, filed on April 2, 2010.

## Claims

1. A photoacoustic imaging apparatus, comprising:
a light source (11);
a plurality of detecting elements (17) arranged in a spatial direction for detecting an acoustic wave (16, 22) generated from a surface of an object (15) and a light absorber (14) inside the object (15), which have absorbed light (12) irradiated from the light source (11), and converting the acoustic wave into detection signals; and
a signal processor (20) for performing Fourier transform, in the spatial direction, on the signals detected by the plurality of detecting elements (17) at the same receiving time so as to obtain spatial frequency signals,
then reducing components exhibiting less than a predetermined frequency from among the spatial frequency signals,
and then performing inverse Fourier-transform on the reduced spatial frequency signals so as to generate image data, wherein
the signal processor performs the inverse Fourier-transform on the resulting spatial frequency signals so as to obtain second signals, and generates the image data using the second signals.

2. The photoacoustic imaging apparatus according to Claim 1, wherein
the signal processor performs Fourier transform after normalizing the detection signals according to an intensity distribution of the irradiated light on the surface of the object.

3. The photoacoustic imaging apparatus according to any one of Claims 1 to 2, wherein
the plurality of detecting elements are two-dimensionally arrayed, and the spatial direction corresponds to one of the dimensions.

4. The photoacoustic imaging apparatus according to any one of Claims 1 to 3, further comprising a member which is disposed between the plurality of detecting elements and the object, and which flattens a surface profile of the object.

5. A photoacoustic imaging method, comprising:
a step of an information processor causing a plurality of detecting elements (17) arranged in a spatial direction to detect an acoustic wave (16, 22) generated from a surface of an object (15) and a light absorber (14) inside the object (15), which have absorbed light (12) irradiated from a light source (11), and converting the acoustic wave (16, 22) into detection signals;
a step of the information processor performing Fourier transform (S201) in the spatial direction on the detection signals detected by the plurality of detecting elements at the same receiving time so as to obtain spatial frequency signals;
a step of the information processor of reducing (S202) components exhibiting less than a predetermined frequency from among the spatial frequency signals; and
a step of the information processor performing inverse-Fourier transform (S203) on the reduced spatial frequency signals so as to generate image data in use of second signals, wherein the step of the information processor performing inverse-Fourier transform includes a step of the information processor performing the inverse Fourier-transform on the resulting spatial frequency signals so as to obtain the second signals and a step of the information processor generating the image data using the second signals.

6. The photoacoustic imaging method according to Claim 5, further comprising a step of the information processor normalizing the detection signals according to an intensity distribution of the irradiated light on the surface of the object before performing Fourier transform.

7. A photoacoustic imaging program for causing an information processor to execute the photoacoustic imaging method according to any of claims 5 and 6.

## Patentansprüche

1. Photoakustische Bildgebungsvorrichtung, mit:
einer Lichtquelle (11);
einer Vielzahl von in einer räumlichen Richtung angeordneten Erfassungselementen (17) zur Erfassung einer akustischen Welle (16, 22), die von einer Oberfläche eines Objekts (15) und einem Lichtabsorber (14) innerhalb des Objekts (15), der das von der Lichtquelle (11) abgestrahlte Licht (12) absorbiert hat, erzeugt wird, und zur Umwandlung der akustischen Welle in ein Erfassungssignal; und
einem Signalprozessor (20) zur Ausführung einer Fourier-Transformation in der räumlichen Richtung an den durch die Vielzahl von Erfassungselementen (17) zur gleichen Empfangszeit erfassten Signalen zur Erhaltung räumlicher Frequenzsignale,
einer anschließenden Verringerung von Komponenten, die weniger als eine vorbestimmte Frequenz aufweisen, aus den räumlichen Frequenzsignalen,
und einer anschließenden Ausführung einer inversen Fourier-Transformation an den verringerten räumlichen Frequenzsignalen zur Erzeugung von Bilddaten, wobei
der Signalprozessor die inverse Fourier-Transformation an den resultierenden räumlichen Frequenzsignalen zur Erhaltung von zweiten Signalen ausführt, und die Bilddaten durch Verwendung der zweiten Signale erzeugt.

2. Photoakustische Bildgebungsvorrichtung gemäß Anspruch 1, wobei
der Signalprozessor eine Fourier-Transformation nach Normalisierung der Erfassungssignale gemäß einer Intensitätsverteilung des abgestrahlten Lichts auf der Oberfläche des Objekts ausführt.

3. Photoakustische Bildgebungsvorrichtung gemäß einem der Ansprüche 1 bis 2, wobei
die Vielzahl von Erfassungselementen zweidimensional angeordnet ist, und die räumliche Richtung einer der Dimensionen entspricht.

4. Photoakustische Bildgebungsvorrichtung gemäß einem der Ansprüche 1 bis 3, ferner mit einem Element, das zwischen der Vielzahl von Erfassungselementen und dem Objekt angeordnet ist, und das ein Oberflächenprofil des Objekts glättet.

5. Photoakustisches Bildgebungsverfahren, mit:
einem Schritt eines Informationsprozessors, der eine Vielzahl von in einer räumlichen Richtung angeordneten Erfassungselementen (17) veranlasst eine akustische Welle (16, 22) zu erfassen, die von einer Oberfläche eines Objekts (15) und einem Lichtabsorber (14) innerhalb des Objekts (15), der das von der Lichtquelle (11) abgestrahlte Licht (12) absorbiert hat, erzeugt wird, und die akustische Welle (16, 22) in ein Erfassungssignal umzuwandeln;
einem Schritt des Informationsprozessors, der (S201) eine Fourier-Transformation in der räumlichen Richtung an den durch die Vielzahl der Erfassungselemente zur gleichen Empfangszeit erfassten Signalen ausführt, um so räumliche Frequenzsignale zu erhalten; und
einem Schritt des Informationsprozessors, der (S202) Komponenten, die weniger als eine vorbestimmte Frequenz aufweisen, aus den räumlichen Frequenzsignalen verringert; und
einem Schritt des Informationsprozessors, der (S203) eine inverse Fourier-Transformation an den verringerten räumlichen Frequenzsignalen zum Erzeugen von Bilddaten unter Verwenden von zweiten Signalen ausführt, wobei der Schritt des Informationsprozessors, der eine inverse Fourier-Transformation ausführt, einen Schritt des Informationsprozessors, der die inverse Fourier-Transformation an den resultierenden räumlichen Frequenzsignalen zum Erhalten der zweiten Signale ausführt, und einen Schritt des Informationsprozessors, der die Bilddaten durch Verwenden der zweiten Signale erzeugt, umfasst.

6. Photoakustisches Bildgebungsverfahren gemäß Anspruch 5, ferner mit einem Schritt des Informationsprozessors, der die Erfassungssignale gemäß einer Intensitätsverteilung des abgestrahlten Lichts auf der Oberfläche des Objekts vor einem Ausführen einer Fourier-Transformation normalisiert.

7. Photoakustisches Bildgebungsprogramm zum Veranlassen eines Informationsprozessors zum Ausführen des photoakustischen Bildgebungsverfahrens gemäß einem der Ansprüche 5 und 6.

## Revendications

1. Appareil d'imagerie opto-acoustique, comprenant :
une source de lumière (11) ;
une pluralité d'éléments de détection (17) disposés dans une direction spatiale, destinés à détecter une onde acoustique (16, 22) générée à partir d'une surface d'un objet (15) et d'un absorbeur de lumière (14) à l'intérieur de l'objet (15), qui ont absorbé une lumière (12) projetée à partir de la source de lumière (11), et à convertir l'onde acoustique en signaux de détection ; et
un processeur de signal (20) destiné à appliquer une transformée de Fourier, dans la direction spatiale, aux signaux détectés par les éléments de la pluralité d'éléments de détection (17) au même instant de réception de façon à obtenir des signaux de fréquence spatiale,
à réduire ensuite des composantes présentant une fréquence inférieure à une fréquence prédéterminée parmi les signaux de fréquence spatiale,
et à appliquer par la suite une transformée de Fourier inverse aux signaux de fréquence spatiale réduite de façon à générer des données d'image, dans lequel
le processeur de signal applique la transformée de Fourier inverse aux signaux de fréquence spatiale ainsi obtenus de façon à obtenir des seconds signaux, et génère les données d'image au moyen des seconds signaux.

2. Appareil d'imagerie opto-acoustique selon la revendication 1, dans lequel
le processeur de signal exécute une transformée de Fourier après une normalisation des signaux de détection conformément à une distribution d'intensité de la lumière projetée sur la surface de l'objet.

3. Appareil d'imagerie opto-acoustique selon l'une ou l'autre des revendications 1 et 2, dans lequel
les éléments de la pluralité d'éléments de détection sont disposés en matrice bidimensionnelle, et la direction spatiale correspond à l'une des dimensions.

4. Appareil d'imagerie opto-acoustique selon l'une quelconque des revendications 1 à 3, comprenant en outre un élément qui est disposé entre les éléments de la pluralité d'éléments de détection et l'objet, et qui aplanit un profil de surface de l'objet.

5. Procédé d'imagerie opto-acoustique, comprenant :
une étape d'un processeur d'informations consistant à amener les éléments d'une pluralité d'éléments de détection (17) disposés dans une direction spatiale à détecter une onde acoustique (16, 22) générée à partir d'une surface d'un objet (15) et d'un absorbeur de lumière (14) à l'intérieur de l'objet (15), qui ont absorbé de la lumière (12) projetée à partir d'une source de lumière (11), et à convertir l'onde acoustique (16, 22) en signaux de détection ;
une étape du processeur d'informations consistant à appliquer une transformée de Fourier (S201) dans la direction spatiale aux signaux de détection détectés par les éléments de la pluralité d'éléments de détection au même instant de réception de façon à obtenir des signaux de fréquence spatiale ;
une étape du processeur d'informations consistant à réduire (S202) des composantes présentant une fréquence inférieure à une fréquence prédéterminée parmi les signaux de fréquence spatiale ; et
une étape du processeur d'informations consistant à appliquer une transformée de Fourier inverse (S203) aux signaux de fréquence spatiale réduite de façon à générer des données d'image lors d'une utilisation de seconds signaux, dans lequel l'étape du processeur d'informations consistant à appliquer une transformée de Fourier inverse comprend une étape du processeur d'informations consistant à appliquer la transformée de Fourier inverse aux signaux de fréquence spatiale ainsi obtenus de façon à obtenir les seconds signaux et une étape du processeur d'informations consistant à générer les données d'image au moyen des seconds signaux.

6. Procédé d'imagerie opto-acoustique selon la revendication 5, comprenant en outre une étape du processeur d'informations consistant à normaliser les signaux de détection conformément à une distribution d'intensité de la lumière projetée sur la surface de l'objet avant d'exécuter une transformée de Fourier.

7. Programme d'imagerie opto-acoustique destiné à amener un processeur d'informations à exécuter le procédé d'imagerie opto-acoustique selon l'une ou l'autre des revendications 5 et 6.
